# EUROPEAN PATENT APPLICATION

(11) **EP 2 385 120 A1**
(43) Date of publication of application: **09.11.2011**
(21) Application number: 10004716.6
(22) Date of filing: 04.05.2010
(51) Int. Cl.: C12N 15/113

(54) **Use of anti-miRNA antisense oligonucleotides for the treatment of pulmonary hypertension**

(71) Applicant: Justus-Liebig- Universitat Giessen, 35390 Giessen (DE); Johann Wolfgang Goethe-Universität Frankfurt am Main, 60325 Frankfurt am Main (DE)
(72) Inventor: Schermuly, Ralf, Prof. Dr., 35794 Mengerskirchen (DE); Grimminger, Friedrich, Prof. Dr., 35510 Butzbach (DE); Dimmeler, Stefanie, Prof. Dr., 60594 Frankfurt (DE); Zeiher, Andreas, Prof. Dr., 60594 Frankfurt (DE)
(74) Representative: Hoppe, Georg Johannes

(57) **Abstract**

The invention pertains to these of an inhibitor molecule that is capable of inhibiting the activity of miRl7, mirR21 or miR92a in a cell for the purpose of treating pulmonary hypertension as well as to a pharmaceutical composition for treating pulmonary hypertension.

## Description

The invention relates to the treatment of pulmonary hypertension.

### Background

Pulmonary hypertension (PH or PHT) is an increase in blood pressure in the pulmonary artery, pulmonary vein, or pulmonary capillaries, together known as the lung vasculature, leading to shortness of breath, dizziness, fainting, and other symptoms, all of which are exacerbated by exertion. Pulmonary hypertension can be a severe disease with a markedly decreased exercise tolerance, eventually leading to heart failure. According to a recent classification, it can be one of five different types: arterial, venous, hypoxic, thromboembolic or miscellaneous (Simonneau G, Galiè N, Rubin LJ, et al. (2004). "Clinical classification of pulmonary hypertension", J. Am. Coll. Cardiol. 43 (12 Suppl S): 5S-12S.)

Pulmonary hypertension is presently considered an incurable disease. Several specific therapies are currently approved which address mostly vasodilatory pathways, but none of these drugs is able to treat pulmonary hypertension effectively. The classes of drugs used in the state of the art include prostanoids, endothelin receptor antagonists, and phosphodiesterase-5 inhibitors

Therefore, it is an object of the present invention to provide a novel means of treating pulmonary hypertension.

MicroRNAs (miRNAs or miRs) are conserved non-coding RNAs which repress protein expression at the post-transcriptional level (He L, Hannon GJ. MicroRNAs: small RNAs with a big role in gene regulation. Nat Rev Genet. 2004; 5: 522-531; Bartel DP. MicroRNAs: genomics, biogenesis, mechanism, and function. Cell. 2004; 116: 281-297; Zhao Y, Srivastava D. A developmental view of microRNA function. Trends Biochem Sci. 2007; 32:189-197), mainly by annealing with the 3' untranslated region of the target mRNA, thus interfering with its translation and/or stability. miRNAs play important roles in the regulation of basic cell functions, including proliferation, differentiation and apoptosis (He L, He X, Lowe SW, Hannon GJ. microRNAs join the p53 network-another piece in the tumour-suppression puzzle. Nat Rev Cancer. 2007; 7:819-822; Wang Y, Medvid R, Melton C, Jaenisch R, Blelloch R. DGCR8 is essential for microRNA biogenesis and silencing of embryonic stem cell self-renewal. Nat Genet. 2007; 39:380-385; Fontana L, Pelosi E, Greco P, Racanicchi S, Testa U, et al. MicroRNAs 17-5p-20a-106a control monocytopoiesis through AML1 targeting and M-CSF receptor upregulation. Nat Cell Biol. 2007; 9:775-787; Felli N, Fontana L, Pelosi E, Botta R, Bonci D, et al. MicroRNAs 221 and 222 inhibit normal erythropoiesis and erythroleukemic cell growth via kit receptor down-modulation. Proc Natl Acad Sci U. S. A. 2005; 102:18081-18086).

Specifically, microRNAs are short (usually with a length of 20 to 24 nucleotides) non-coding RNAs that are involved in post-transcriptional regulation of gene expression in multicellular organisms by affecting both the stability and translation of mRNAs. miRNAs are transcribed by RNA polymerase II as part of capped and polyadenylated primary transcripts (pri-miRNAs) that can be either protein-coding or non-coding. The primary transcript is cleaved by the Drosha ribonuclease III enzyme to produce an approximately 70-nt stem-loop precursor miRNA (pre-miRNA), which is further cleaved by the cytoplasmic Dicer ribonuclease to generate the mature miRNA and antisense miRNA star (miRNA*) products. The mature miRNA is incorporated into a RNA-induced silencing complex (RISC), which recognizes target mRNAs through imperfect base pairing with the miRNA and most commonly results in translational inhibition or destabilization of the target mRNA.

### Description of the Invention

The present invention provides a means for treating pulmonary hypertension. The term "pulmonary hypertension" includes groups 1 to 4 according the updated clinical classification (Group 1: Pulmonary arterial hypertension (PAH); Group 2: Pulmonary hypertension with left heart disease; Group 3: Pulmonary hypertension associated with lung diseases and/or hypoxemia; Group 4: Pulmonary hypertension owing to chronic thrombotic and/or embolic disease) (Updated clinical classification of pulmonary hypertension. Simonneau G, Robbins IM, Beghetti M, Channick RN, Delcroix M, Denton CP, Elliott CG, Gaine SP, Gladwin MT, Jing ZC, Krowka MJ, Langleben D, Nakanishi N, Souza R. J Am Coll Cardiol. 2009;54 (Suppl): S43-54).

The terms "treating" or "treatment" refer to the clinical improvement of the disease, to curing the disease and/or to stopping or slowing down of the progression of the disease.

The terms miR17, mirR21 and miR92a refer to both processed (mature) miRs as well as to precursor molecules thereof and are sometimes referred to as miR molecule herein.

Specifically, the term "miR17" refers to both miR17-5p and miR17-3p, and the term "miR92a" refers to both precursor molecules (pre92a-1, pre-92a-2) and to the processed molecule (miR92a), as it is also possible to inhibit the function of miR92a also on the level of its precursor molecules.

Sequences of the human miR molecules described herein are given as SEQ ID 1 (miRBAse (http://mirbase.org/) ID: hsa-mir-17; miRBAse accession number: MI0000071), SEQ ID NO. 2 (miRBAse ID: hsa-miR-17; miRBAse accession number: MIMAT0000070), SEQ ID NO. 3 (miRBAse ID: hsa-mir-21; miRBAse accession number: MI0000077), SEQ ID NO. 4 (miRBAse ID: hsa-miR-21; miRBAse accession number: MIMAT0000076;), SEQ ID NO. 5 (miRBAse ID: hsa-mir-92a-1; miRBAse accession number: MI0000093), and SEQ ID NO. 6 (miRBAse ID: hsa-miR-92a; miRBAse accession number: MIMAT0000092).

As far as the invention relates to organisms other than humans, the respective miR sequences for these organisms can be obtained from known databases or determined using methods known to a person of skill in the art.

### Use of an inhibitor molecule for treating pulmonary hypertension

In one aspect, the invention pertains to the use of an inhibitor molecule (antagonist), such as a nucleic acid molecule (preferably a single stranded oligonucleotide), that inhibits the activity of miR17, mirR21 and/or miR92a in a cell, either through degradation or functional inhibition of the respective miR, for the purpose of treating pulmonary hypertension.

The inhibition of the activity of miR17, mirR21 and/or miR92a can be direct or indirect. An example for a direct inhibition is the interaction of a miR molecule with an antisense RNA (i.e. with a RNA that has a reverse complementary sequence to the miR molecule), thereby forming a duplex, which leads to the degradation of the miR molecule. An example for an indirect inhibition is the inhibition of a protein that is involved in the processing of a miR molecule.

Inhibition of the activity of miR17, mirR21 and/or miR92a in the context of the invention can be achieved in a preferred embodiment by any substance that is able to inhibit miR-17, miR-21 and/or miR-92a either by inhibiting the synthesis/processing or by inhibiting the silencing function of the microRNA. Thus, any compound interfering with the microRNA pathway, for example by inhibiting the function of the proteins Pasha, Drosha, Dicer or Argonaut family proteins can be used to inhibit the activity of miR17, mirR21 and/or miR92a according to the invention.

Furthermore, a compound inhibiting the expression of the precursor microRNA of miRNA-17, such as, for example inhibitors of polymerase II or III, are (indirect) inhibitors of miRNA-17 expression. The mature miR also serves as a target for the design of inhibitors of miR function. Nucleic acids having perfect or mismatched complementarity to the microRNA may be used to inhibit, or to compete with the binding of the endogenous miR with its target mRNA.

Since miRs target mRNA on the basis of Watson-Crick base-pairing, it is preferred that the inhibition of miRs is performed through an antisense oligonucleotide, which is reverse complementary to the miR and basepairs with the miR in competition with the endogenous mRNA target.

The inhibition of the activity of miR17, mirR21 and/or miR92a can be achieved using a molecule chosen from the group consisting of antisense DNA-oligonucleotides, antisense RNA-oligonucleotides, antisense DNA-RNA-oligonucleotides, an antisense 2'-O-methyl oligoribonucleotides, antisense oligonucleotides containing phosphorothiaote linkages, antisense oligonucleotides containing Locked Nucleic Acid LNA^{®} bases, morpholino antisense oligos, antagomirs, and mixtures thereof.

Moreover, particularly preferred inhibitor molecules are antisense oligonucleotides which are chemically modified to improve the thermal stability of the duplex between the antisense oligonucleotide and the miR, such as LNA^{®}-antimiRs. Other preferred chemical modified oligonucleotides include morpholinos, 2'-O-methyl, 2'-O-methoxyethyl oligonucleotides and cholesterol-conjugated 2'-O-methyl modified oligonucleotides (antagomirs). Combinations of the above modifications can also occur in the inhibitor molecule. The chemically modified inhibitor molecules antagomir-17 (SEQ ID NO 10), antagomir-21 (SEQ ID NO 11), and antagomir-92a (SEQ ID NO 12) are particularly preferred inhibitor molecules.

As known to a person of skill in the art, LNA^{®} is a modified RNA nucleotide. The ribose moiety of an LNA nucleotide is modified with an extra bridge connecting the 2' oxygen and 4' carbon. This bridge locks the ribose in the 3'-endo conformation. LNA nucleotides can be mixed with DNA or RNA bases in the oligonucleotide whenever desired.

Morpholino oligonucleotides comprise bases that are bound to morpholine rings instead of deoxyribose rings and linked through phosphorodiamidate groups instead of phosphates.

In one preferred embodiment of the invention, the nucleic acid molecule that inhibits the activity of miR17, mirR21 and/or miR92a is perfectly reverse-complementary in sequence to miR17, mirR21 or miR92a, comprising a mispairing base at the cleavage site of Ago2 and/or a base modification to inhibit Ago2 cleavage, such as a 2'-O-methyl group.

If the inhibitor molecule is a nucleic acid, it is preferably an RNA. The RNA may or may not comprise chemical modifications, e.g. a 2' O-methyl group, as described above and herein. The inhibitory nucleic acids preferably have a length of 8 to 24 nucleotides, preferably of 15 to 22 nucleotides.

Preferred are antisense-17 (SEQ ID NO. 7), antisense-21 (SEQ ID NO. 8), and antisense-92 (SEQ ID NO. 9) as inhibitor molecules. Good results have also been achieved using inhibitor molecules with a partial sequence of SEQ ID NOs. 7, 8, and 9, wherein 8 to 21 nucleotides are present.

Other preferred inhibitory molecules are antagomirs such as the RNA molecules with the sequences of SEQ ID NOs. 7 to 9, further comprising chemical modifications, namely: 3' cholesterol, 2' O-methyl, four consecutive phosphothiolate groups from the 3' side of the molecule, and two consecutive phosphothiolate groups from the 3' side of the molecule (according to Krützfeldt et al., Nature, 2005; 438: 685-689.).

These inhibiting molecules (antagomirs) are here described as SEQ ID NO. 10 (against miR17, antagomir-17), SEQ ID NO. 11 (against miR21, antagomir-21), and SEQ ID NO. 12 (against miR92a, antagomir-92a).

In a further embodiment, inhibitor molecules with a sequence of SEQ ID NOs. 7 to 9 and with a LNA component have also shown good results in inhibiting mirR17, miR21 and miR92a, respectively.

Methods of synthesizing such nucleic acids, such as antagomirs, are known to a person of skill in the art.

According to the present invention, a single inhibitor molecule for inhibiting the activity of mirR21, miR17, and/or miR92a can be used. It is also possible to use a combination of two or more inhibitor molecules that inhibit at least one of mirR21, miR17, and miR92a. Preferably, more than one inhibitor molecules is used against at least two of mirR21, miR17, and/or miR92a. In a preferred embodiment of the invention, at least one inhibitor molecules against each of mirR21, miR17, and miR92a is used simultaneously or consecutively.

In a preferred embodiment of the invention, an inhibitor molecule against miR21 is used together with an inhibitor molecule against miR17. It is particularly preferred that an antagomir21 is used together with an antagomir17, most preferred the antagomir21 according to SEQ ID NO. 11 with the antagomir17 according to SEQ ID NO. 10.

Introduction of the inhibitor molecule into a cell or cells can be done using physical methods such as microinjection or electroporation, using chemical methods such as potassium phosphate, or through viruses. When the inhibiting molecule is an RNA, it is also possible to introduce a nucleic acid into the cell, e.g. as part of a vector such as a plasmid or virus, from which an inhibitor molecule in the form of an RNA is transcribed. Such methods are known to a person of skill in the art.

The inhibition of activity of miR17, mirR21 and/or miR92a in a cell can refer to the reduction of the number of molecules in the cell and therefore can be assessed by measuring the expression of miRNA-17, miR-21 and miR-92a using Northern blotting or RT-PCR or any other of the multiple techniques for the identification and quantification of microRNAs known in the art. In addition to Northern blotting and RT-PCR, assessing microRNA expression can, for example, be performed by means of microRNA expression arrays, fluorescent nucleic acid probes, for example coupled to membranes or beads, and antibody based detection systems.

In an indirect approach, the activity of the microRNA can further be measured by in vitro or in vivo reporter assays. For example, a person of skill in the art could without harnessing inventive skill design reporter assays based on a so-called "seed sequence" of the miRNAs (such as a sequence according to SEQ ID NOs. 2, 4, and 6) that allow for an easy screening of candidate miR inhibitor molecules. In such an approach, the target-sequence of the miR could be introduced into the 3' or 5' untranslated regions of a reporter gene of choice. This miR-sensitive construct is then transformed into a suitable cell expression system, which is subsequently brought into contact with the candidate compound. The activity of the reporter gene in samples that were contacted with the compound in comparison with the activity of the reporter gene in control samples gives information about the inhibitory effect of the candidate compound.

In yet another embodiment, the inhibitory action of an inhibitor molecule of miR-17 comprises assessing the activity of a gene regulated by miRNA-17 such as, for example, the gene for p21.

### Pharmaceutical composition

In another aspect, the invention pertains to a pharmaceutical composition comprising an inhibitor of miR-17, miR-21 and/or miR-92a, as described above and herein.

In a preferred embodiment, the pharmaceutical composition comprises an inhibitor molecule (antagonist) of miR-17, miR-21 and/or miR- 92a activity that is selected from the group consisting of antisense DNA- and/or RNA-oligonucleotides, antisense 2'-O-methyl oligoribonucleotides, antisense oligonucleotides containing phosphorothiaote linkages, antisense oligonucleotides containing Locked Nucleic Acid (LNA^{®}) bases, morpholino antisense oligos, antagomirs, and mixtures thereof. Particularly preferred is the presence of an antagomir of miR-17, miR-21 and/or miR-92a.

In a particular preferred embodiment of the pharmaceutical composition, the antagonist of miRNA-17 comprises a sequence that is complementary to the mature sequence of miRNA-17, or combinations thereof.

In another aspect, the invention also pertains to the use of an inhibitor molecule, such as a nucleic acid, that inhibits the activity of miR17, mirR21 or miR92a in a cell for manufacturing a pharmaceutical composition for treating pulmonary hypertension.

### Method for treating a patient suffering from pulmonary hypertension

In a further aspect, the invention pertains to a method for treating a patient suffering from pulmonary hypertension, comprising introducing a nucleic acid molecule that inhibits the activity miR17, mirR21 and/or miR92a into a cell.

It is preferred that the patient is a mammal, in particular a human. Accordingly, the cell in which the inhibitor molecule inhibits the activity of miR17, mirR21 and/or miR92a is preferably a mammalian cell, most preferably a human cell, in particular a vascular cell of the lung. The cell can be part of an organism, organ or tissue, or it can be a single cell, e.g. in a tissue culture. Moreover, the cell can be treated *ex vivo* or *in vivo.*

The pharmaceutical composition of the invention may additionally comprise a pharmaceutically acceptable carrier, diluent, and/or adjuvant.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Powders can contain, in addition to a compound of this invention, excipients such as lactose, talc, silicic acid, aluminum hydroxide, calcium silicates and polyamide powder, or mixtures of these substances.

These pharmaceutical compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents that delay absorption such as aluminum monostearate and gelatin.

The present invention therefore includes pharmaceutical formulations comprising the nucleic acids described herein, including pharmaceutically acceptable salts thereof, in pharmaceutically acceptable carriers for aerosol, oral and parenteral administration. Also, the present invention includes such compounds, or salts thereof, which have been lyophilized and which may be reconstituted to form pharmaceutically acceptable formulations for administration, as by intravenous, intramuscular, or subcutaneous injection. Administration may also be intradermal or transdermal.

In accordance with the present invention, a nucleic acid described herein, and pharmaceutically acceptable salts thereof, may be administered orally or through inhalation as a solid, or may be administered intramuscularly or intravenously as a solution, suspension or emulsion. Alternatively, the compounds or salts may also be administered by inhalation, intravenously or intramuscularly as a liposomal suspension. Pharmaceutical formulations are also provided which are suitable for administration as an aerosol, by inhalation. These formulations comprise a solution or suspension of the desired nucleic acid herein, or a salt thereof, or a plurality of solid particles of the compound or salt. The desired formulation may be placed in a small chamber and nebulized. Nebulization may be accomplished by compressed air or by ultrasonic energy to form a plurality of liquid droplets or solid particles comprising the compounds or salts. The liquid droplets or solid particles should have a particle size in the range of about 0.5 to about 5 µm. The solid particles can be obtained by processing the solid compound of any nucleic acid described herein, or a salt thereof, in any appropriate manner known in the art, such as by micronization. Most preferably, the size of the solid particles or droplets will be from about 1 µm to about 2 µm. In this respect, commercial nebulizers are available to achieve this purpose.

Preferably, when the pharmaceutical formulation suitable for administration as an aerosol is in the form of a liquid, the formulation will comprise a water-soluble compound of any nucleic acid described herein, or a salt thereof, in a carrier that comprises water. A surfactant may be present that lowers the surface tension of the formulation sufficiently to result in the formation of droplets within the desired size range when subjected to nebulization.

Peroral compositions also include liquid solutions, emulsions, suspensions, and the like. The pharmaceutically acceptable carriers suitable for preparation of such compositions are well known in the art. Typical components of carriers for syrups, elixirs, emulsions, and suspensions include ethanol, glycerol, propylene glycol, polyethylene glycol, liquid sucrose, sorbitol, and water. For a suspension, typical suspending agents include methyl cellulose, sodium carboxymethyl cellulose, tragacanth, and sodium alginate; typical wetting agents include lecithin and polysorbate 80; and typical preservatives include methyl paraben and sodium benzoate. Peroral liquid compositions may also contain one or more components such as sweeteners, flavoring agents and colorants disclosed above.

Pharmaceutical compositions may also be coated by conventional methods, typically with pH or time-dependent coatings, such that the subject compound is released in the gastrointestinal tract in the vicinity of the desired topical application, or at various times to extend the desired action. Such dosage forms typically include, but are not limited to, one or more of cellulose acetate phthalate, polyvinylacetate phthalate, hydroxypropyl methyl cellulose phthalate, ethyl cellulose, waxes, and shellac.

Other compositions useful for attaining systemic delivery of the nucleic acids include sublingual, buccal and nasal dosage forms. Such compositions typically comprise one or more of soluble filler substances such as sucrose, sorbitol and mannitol; and binders such as acacia, microcrystalline cellulose, carboxymethyl cellulose and hydroxypropyl methyl cellulose. Glidants, lubricants, sweeteners, colorants, antioxidants and flavoring agents disclosed above may also be included.

Means of oral delivery have been described e.g. in Aourdi et al., Nature 458, 1180-1184, 2009.

The pharmaceutical compositions of this invention can also be administered topically to a subject, e.g., by the direct laying on or spreading of the composition on the epidermal or epithelial tissue of the subject, or transdermally via a patch. Such compositions include, for example, lotions, creams, solutions, gels and solids. These topical compositions preferably comprise an effective amount, usually at least about 0.1 %, and preferably from about 1 % to about 5 %, of a nucleic acid of the invention. Suitable carriers for topical administration preferably remain in place on the skin as a continuous film, and resist being removed by perspiration or immersion in water. Generally, the carrier is organic in nature and capable of having dispersed or dissolved therein the therapeutic compound. The carrier may include pharmaceutically acceptable emolients, emulsifiers, thickening agents, solvents and the like.

Appropriate dosage levels may be determined by any suitable method known to one skilled in the art. It will be understood, however, that the specific dose level for any particular patient will depend upon a variety of factors including the activity of the specific compound employed, the age, body weight, general health, sex, diet, time of administration, route of administration, rate of excretion, drug combination and the severity of the condition to be treated. Dosage levels preferably lie in the range of 0.01 to 100 mg/kg body weight, more preferably in the range of 0.1 to 50 mg/kg body weight of the patient to be treated.

For a pharmaceutical composition of the invention that comprises more than one active agent, the respective active agents may be formulated together in a single dosage form. Alternatively, they may be formulated separately and packaged together, or they may be administered independently. In certain cases, a patient may be receiving one drug for the treatment of another indication; this invention then comprises administering the other drug.

It may be advantageous to combine or co-administer a pharmaceutical composition of the invention with other classes of drug. Drugs which may be co-administered with a nucleic acid of the invention include, but are not limited to vasodilatory drugs like calcium channel blockers, prostanoids, phosphodiesterase 5 inhibitors, and endothelin receptor antagonists. The respective drugs may be administered simultaneously, separately or sequentially.

In one embodiment, the present invention does not refer to methods for treatment of the human or animal body by therapy or to diagnostic methods practiced on the human or animal body.

### Examples

### Materials and Methods

### Animals and Experimental design

### Hypoxia-induced PH in mice

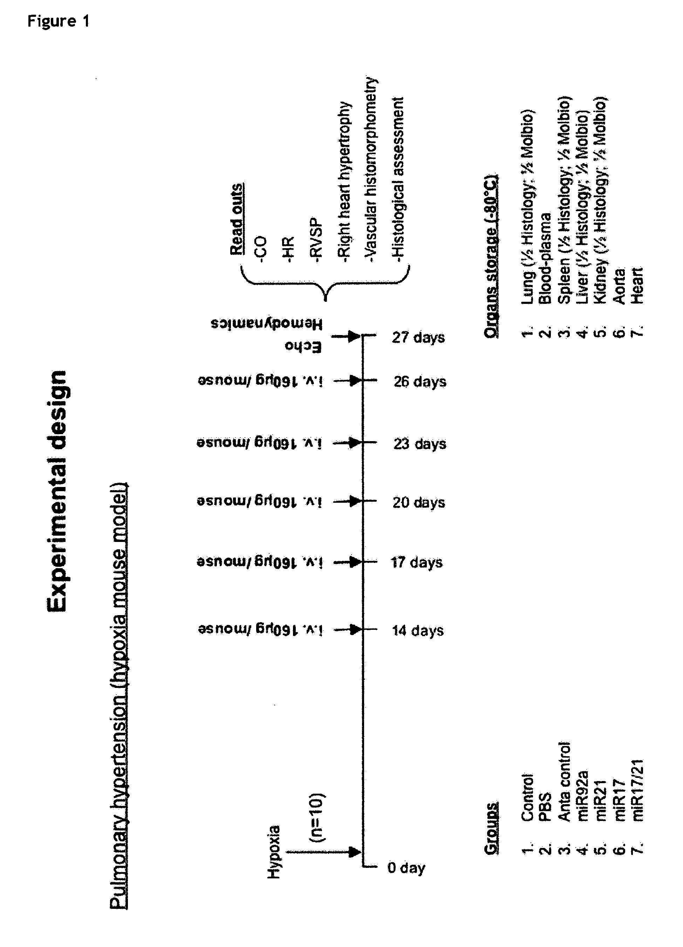

Adult C57B1/6J mice were obtained from Charles River Laboratories. Animals were housed under controlled temperature (22 °C) and lighting (12/12-hour light/dark cycle), with free access to food and water. The animals were exposed to chronic hypoxia (10 % O₂) in a ventilated chamber. The level of hypoxia was held constant by an auto regulatory control unit (model 4010, O₂ controller, Labotect; Göttingen, Germany) supplying either nitrogen or oxygen. Excess humidity in the recirculating system was prevented by condensation in a cooling system. CO₂ was continuously removed by soda lime. Cages were opened once a day for cleaning as well as for food and water supply. The chamber temperature was maintained at 22-24 °C. For antagomir treatment, mice were exposed to normoxia and hypoxia for 28 days. In addition, hypoxia exposed mice were divided into 6 groups and treated with inhibitor molecules in the form of antagomirs of miR 17 (SEQ ID NO. 10) miR 21 (SEQ ID NO. 11), miR17/21 (SEQ ID NOs. 10, 11), miR 92a (SEQ ID NO. 12), antagomir control and placebo (PBS). All groups of mice treated intravenously (tail vein) day 14, day 17, day 20, day 23, and day 26 with antagomirs of miR 17 (SEQ ID NO. 10), miR 21 (SEQ ID NO. 11), miR17/21 (SEQ ID NOs. 10, 11), miR 92a (SEQ ID NO. 12), antagomir control and placebo (PBS) (**Figure 1**).

### MCT-induced PH in rats

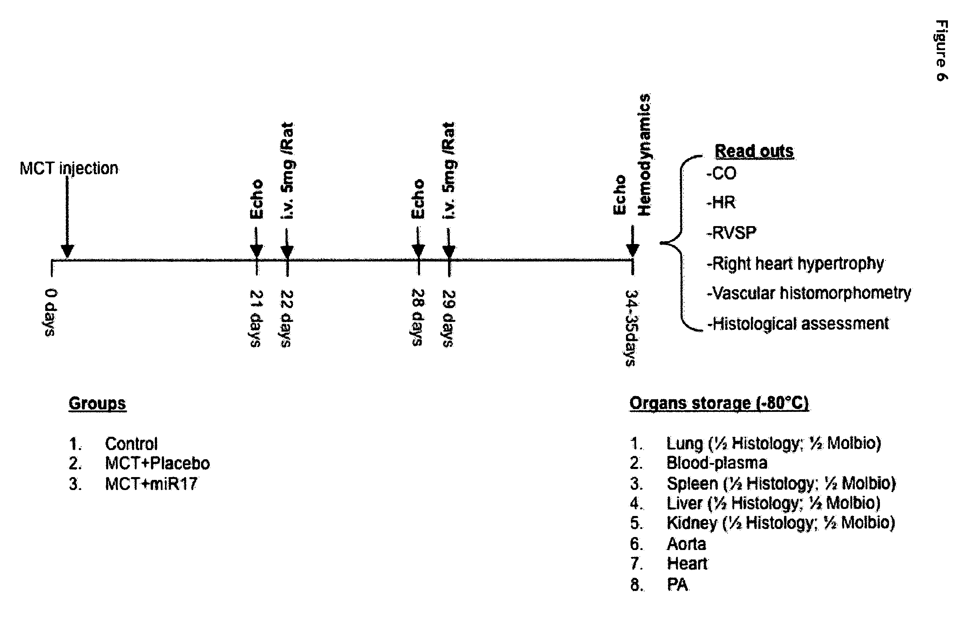

Adult male Sprague-Dawley rats (300-350 g in body weight; Charles River Laboratories, Sulzfeld, Germany) were given s.c. injection of saline or monocrotaline (MCT) (60 mg/kg BW). After 21 days, MCT-injected rats were treated intravenously (tail vein) on day 22 and day 29 with antagomirs of miR 17 (SEQ ID NO. 10) or antagomir control (placebo). Echocardiographic measurement of acceleration time (AcT), cardiac output (CO), heart rate (HR), right ventricular dimensions (RVD) and right ventricular wall thickness (RVWT) was performed at 21st, 28th and 35th day after monocrotaline injection for all experimental groups (**Figure 6**). At the end of experiments, rats were sacrificed for hemodynamic and right heart hypertrophy (RV/(LV+S) ratio) measurement.

### Hemodynamic and Right Ventricular Hypertrophy (RVH) Measurements

### Hypoxia-induced PH in mice

Mice were anaesthetized with intraperitoneal injection ketamine (6 mg/100 g BW) and xylazine (1 mg/100 g BW) combination. The trachea was cannulated, and the lungs were ventilated with room air at a tidal volume of 0.2 ml and a rate of 120 breaths per minute. Systemic arterial pressure (SAP) was determined by placing a catheter into the carotid artery. For measurement of right ventricular systolic pressure (RVSP), a catheter was inserted into the RV via the right jugular vein. The catheters were connected to fluid-filled pressure transducers and the pressure measurements were recorded as described previously (Schermuly RT et al, "Reversal of experimental pulmonary hypertension by PDGF inhibition". J Clin Invest. 2005; 115: 2811-21). After recording SAP and RVSP, the animals were exsanguinated and the lungs were flushed with sterile saline to get rid of blood. The right lung was removed and snap frozen in liquid nitrogen. The left lung was then perfused with neutral buffered formalin (3.5 %, pH 7.4) through pulmonary artery and with saline through trachea at a constant pressure of 22 and 11 cm H₂O respectively and was processed for histochemistry. The hearts were isolated and the right ventricle (RV) was separated from the left ventricle and septum (LV + S) under dissection microscope. These dissected hearts were weighed to obtain the right to left ventricle plus septum ratio (RV/LV+S) as a measure for right ventricular hypertrophy as described previously (Schermuly RT et al, "Reversal of experimental pulmonary hypertension by PDGF inhibition". J Clin Invest. 2005; 115: 2811-21). As an alternative measurement for the RV hypertrophy, the RV to body weight ratio (RV/BW) was also analyzed.

### MCT-induced PH in rats

For monitoring hemodynamics, the animals were initially anesthetized i.p. with ketamine and domitor. Rats were tracheotomized and artificially ventilated at a constant frequence of 60 breaths per minute with inspiratory flow rate of 500-600 cc/min. The left carotid artery was isolated and cannulated with a polyethylene cannula connected to a fluid-filled force transducer and the systemic arterial pressure (SAP) was measured. A catheter was inserted through the right jugular vein into the right ventricle for measurement of right ventricular systolic pressure (RVSP). Cardiac output was calculated using the Fick principle, by employing the mixed venous oxygen and the arterial oxygen content as previously described (Schermuly RT et al, "Reversal of experimental pulmonary hypertension by PDGF inhibition". J Clin Invest. 2005; 115: 2811-21). Arterial and mixed venous samples were collected (150 µL) and analyzed for partial pressure of oxygen, pH and carbon dioxide tension (ABL330; Radiometer, Copenhagen, Denmark). Hemoglobin and oxygen saturation were measured using an OSM2 hemoximeter (Radiometer). Total pulmonary resistance (TPR) and total systemic resistance 3 (TSR) indexed to the body weight were determined as described previously. The animals were ex-sanguinated and the lungs were flushed with sterile saline to get rid of blood. The left lung was fixed for histology in 3.5 % neutral buffered formalin and the right lung was snap frozen in liquid nitrogen. The heart was isolated and dissected under microscope. The right ventricular wall was separated from the left ventricular wall and ventricular septum. Dry weight of the right ventricle, free left ventricular wall and ventricular septum was determined. Right ventricular hypertrophy was expressed as the ratio of weight of the right ventricular wall (RV) and that of the free left ventricular wall and ventricular septum (LV+S).

### High-resolution ultrasound biomicroscopy (UBM)

Anesthesia was induced with 3 % isoflurane gas and maintained with 1.0 - 1.5 % isoflurane in room air supplemented with 100 % O₂. Animals were laid supine on a heating platform with all legs taped to ECG electrodes for heart rate (HR) monitoring. Body temperature was monitored via a rectal thermometer (Indus Instruments, Houston, TX) and maintained at 36.5-37.5 °C using a heating pad and lamp. The chest of the rats and mice was shaved and treated with a chemical hair remover to reduce ultrasound attenuation. To provide a coupling medium for the transducer, a pre-warmed ultrasound gel was spread over the chest wall. Transthoracic two-dimensional, M-mode and Doppler imaging were performed with VisualSonics Vevo770 high-resolution imaging system equipped by 25-MHz and 30-MHz transducer (VisualSonics, Toronto, Canada) 2 weeks after treatment. All studies were performed by an experienced sonographer who was blinded to results of invasive and morphometric studies.

### M-mode

RV free wall thickness (RVWT) was measured in the modified parasternal long-axis view. RV outflow tract (RVOT) dimension were measured from the RVOT view at the level of the aortic valve. For determine TAPSE M-mode cursor was oriented to the junction of the tricuspid valve plane with the RV free wall using the apical four chamber view.

### Pulsed wave Doppler

Cardiac output was calculated as the product of the velocity-time integral of the pulsed-Doppler tracing in the LV outflow tract, the cross-sectional area of the LV outflow tract, and the HR. Pulmonary artery acceleration time (PAAT) was measured from the pulsed-wave Doppler flow velocity profile of the RV outflow tract in the parasternal short-axis view and defined as the interval from the onset to the maximal velocity of forward flow. All echocardiographic parameters were calculated off-line using tool section of the Visual Sonics Vevo770 system.

### Histology and pulmonary vascular morphometry

The formalin-fixed lungs were subject to paraffin embedding. The paraffin-embedded tissues were subject to sectioning to yield 3 µm thick sections. Elastica staining was performed according to common histopathological procedures. The degree of muscularization of small peripheral pulmonary arteries was assessed by double-staining the 3 µm sections with an anti-α -smooth muscle actin antibody (dilution 1:900, clone 1A4, Sigma, Saint Louis, Missouri) and antihuman von Willebrand factor antibody (vWF, dilution 1:900, Dako, Hamburg, Germany) followed by analysis of the vessels using a computerized morphometric analysis system (QWin; Leica, Wetzlar, Germany) to determine the degree of pulmonary artery muscularization. In each rat, 80 to 100 intra-acinar arteries (25 to 50 µm diameter) were categorized as muscular, partially muscular, or non-muscular. Arteries of the same size were 1 additionally analyzed for the medial wall thickness as previously described. In mouse sections, 80-100 intra-acinar vessels at a size between 20 and 70 µm accompanying either alveolar ducts or alveoli were analyzed. Each vessel was categorized as described for rats. Additionally, arteries of the same size were analyzed to calculate the medial wall thickness, and 150 - 200 vessels were included in the analysis. All analyses were done in a blinded fashion (Schermuly RT et al, "Reversal of experimental pulmonary hypertension by PDGF inhibition". J Clin Invest. 2005; 115: 2811-21).

### Immunohistochemistry

Paraffin-embedded lung tissue sections with thickness of 3 µm were deparaffinized in xylene and rehydrated in a graded ethanol series to phosphate-buffered saline (PBS, pH 7.4). Antigen retrieval was performed by a combination of pressure cooking in citrate buffer (pH 6.0) and enzymatic treatment with trypsin. The sections were pretreated with hydrogen peroxide (15 %) to quench endogenous peroxidase activity. Following blocking with BSA (10 %) for one hours and then with blocking serum (Impress reagent kit, Vector Laboratories, CA) for 20 minutes, the sections were incubated with primary antibodies. Murine lung tissue sections were incubated with BMP-RII antibody (Santa Cruz), Integrin antibody (Santa Cruz), Smad 1-5-8 antibody (Santa Cruz), and TGFß-RII antibody (Santa Cruz) at 4 °C overnight. Development of the dye was carried out with peroxidase and substrate (NovaRed substrate kit, Vector Laboratories, CA) according to manufacturer's instruction (Vector laboratories, CA). Finally, sections were counterstained with hematoxylin (Zymed laboratory, UK) and coverslipped using mounting medium.

### Data analysis

All data were expressed as Mean ± SEM. The different experimental groups were analyzed by one-way ANOVA and Newman-Keuls post-hoc test for a multiple comparisons. A values of p<0.05 (*), p<0.01 (**) and p<0.001 (***) were considered as statistically significant.

### Results

### Hypoxia-induced PH in mice

Effects of miR 17 antagomirs (SEQ ID NO. 10), miR 21 antagomirs (SEQ ID NO. 11), miR17/21 antagomirs (SEQ ID NOs. 10, 11) and miR 92a antagomirs (SEQ ID NOs. 12) on hemodynamics

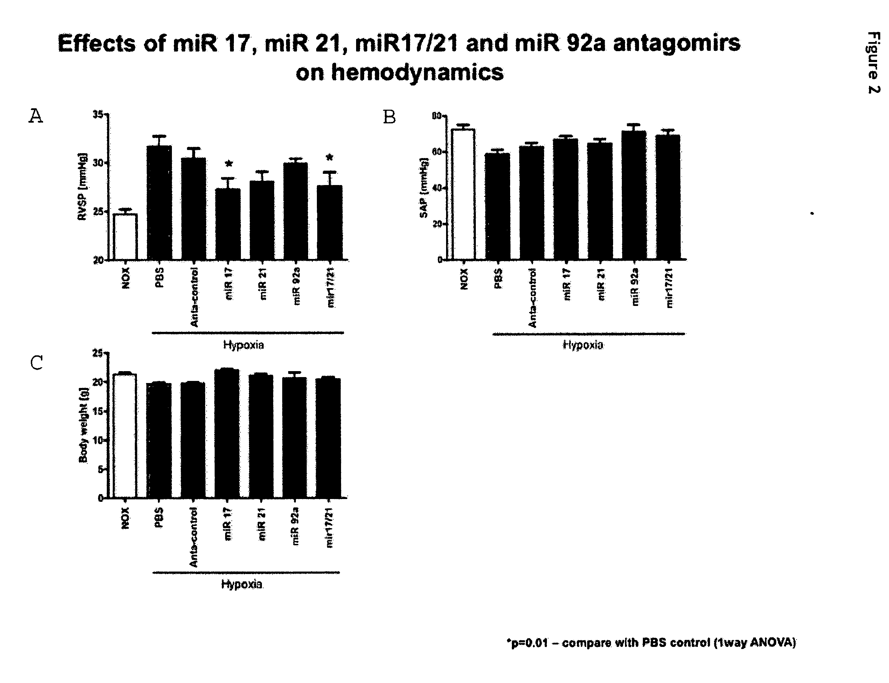

In mice, chronic hypoxic exposure resulted in a significant increase in RVSP (PBS; 31.7 ± 1.1 and Anta-control; 30.4 ± 1.0 versus 24.7 ± 0.5 mmHg under normoxia) (**Figure 2A**). Chronic treatment with miR 17 and miR 17/21 from day 14 to 28 of hypoxic exposure resulted in a significant decrease in RVSP (miR 17; 27.25 ± 1.17 and miR 17/21; 27.57 ± 1.445). In addition, miR 21 and miR 92a antagomirs from day 14 to 28 of hypoxic exposure did not result in a significant decrease in RVSP (miR 21; 28.06 ± 1.01 and miR 92a; 29.88 ± 0.55 mmHg respectively). No significant changes in SAP and bodyweight were noted among the experimental groups (**Figure 2B****, C**).

Effects of miR 17 antagomirs (SEQ ID NO. 10), miR 21 antagomirs (SEQ ID NO. 11), miR17/21 antagomirs (SEQ ID NOs. 10, 11) and miR 92a antagomirs (SEQ ID NOs. 12) on right heart hypertrophy

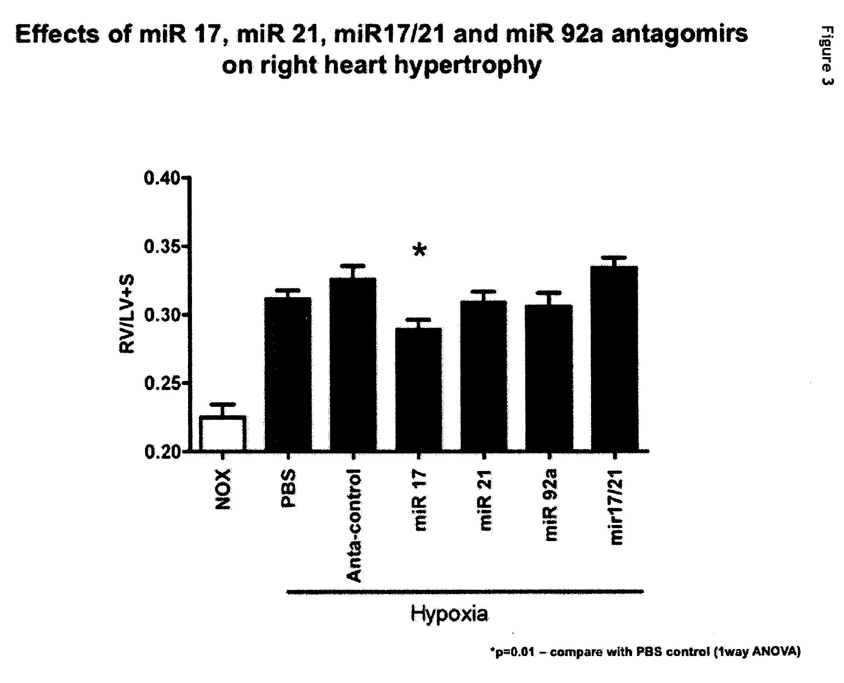

The increased RVSP was accompanied by development of RV hypertrophy in chronic hypoxia mice as evident from a significant increase in the ratio of RV to LV plus septum weight [RV/(LV+S)] in anta-control group (0.326 ± 0.009) as compared to that in normoxia control (0.22 ± 0.009) **(****Figure 3****)**. Chronic treatment with miR 17, miR 21 and miR 92a tendency towards reduction in RV hypertrophy was observed. Among them, miR 17 regimens exhibited a significant reduction in RV/(LV+S) (0.289 ± 0.007). No significant reduction in RV/(LV+S) was noted in the miR 21, miR 17/21 and miR 92a groups (miR 21; 0.31 ± 0.008, miR 17/21; 0.33 ± 0.0074 and miR 92a; 0.30 ± 0.0098).

Effects of miR 17 antagomirs (SEQ ID NO. 10), miR 21 antagomirs (SEQ ID NO. 11), miR17/21 antagomirs (SEQ ID NOs. 10, 11), and miR 92a antagomirs (SEQ ID NOs. 12) on acceleration time (AcT) and cardiac output (CO)

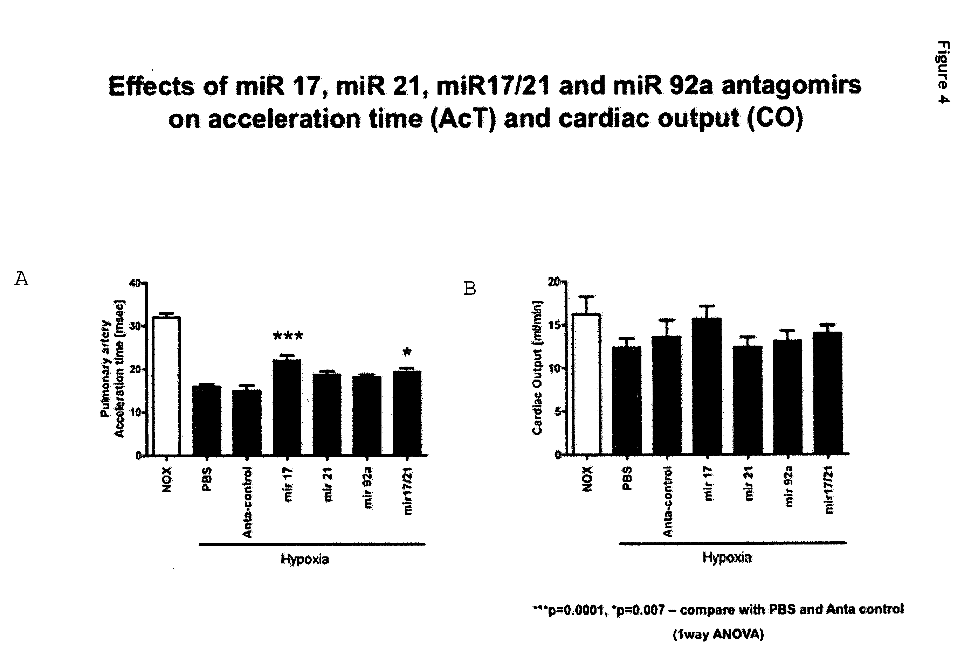

Chronic hypoxia induced a significant decrease of AcT in Anta-control group (15.01 ± 1.124), as compared with normoxia (32 ± 0.935). Treatment of the mice with miR 17 (22 ± 1.225) and miR 17/21 (19.38 ± 0.78) resulted in a noticeably increased AcT in comparison with Anta-control group. No significant changes were observed in miR 92a group (18.13 ± 0.625) and miR 21 group (18.75 ± 0.721) (**Figure 4A**). However, no significant changes were observed in CO in normoxia control (16.21 ± 2.07) and chronic hypoxia treatment groups with miR 17 (15.70 ± 1.45), miR 21 (12.41 ± 1.22) miR 17/21 (13.94 ± 0.98), and miR 92a (13.13 ± 1.16) as compared to Anta-control group (13.64 ± 1.9) **(****Figure 4B****).**

Effects of miR 17 antagomirs (SEQ ID NO. 10), miR 21 antagomirs (SEQ ID NO. 11), miR17/21 antagomirs (SEQ ID NOs. 10, 11) and miR 92a antagomirs (SEQ ID NOs. 12) on pulmonary vascular remodeling

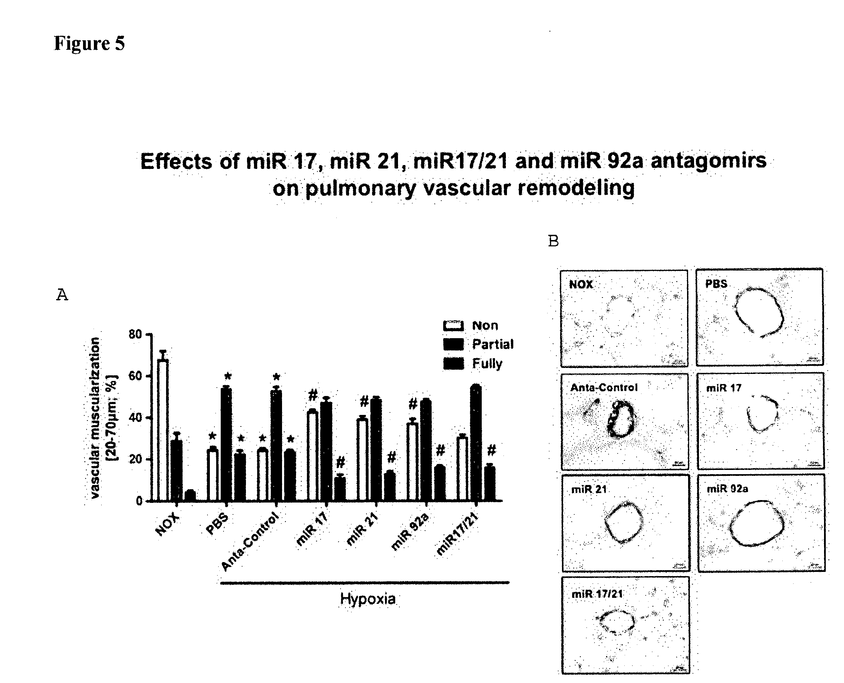

Quantification of the degree of muscularization was performed in pulmonary arteries with a diameter between 20 to 70 µm. In control animals, the majority of vessels between 20 to 70 µm are usually non- and partially muscularized. In the hypoxia exposed control animals, both in PBS and in Anta-control groups, a significant decrease in non-muscularized pulmonary arteries occurred with a concomitant increase in fully and partially muscularized pulmonary arteries (**Figure 5A**). Chronic treatment with miR 17, miR 21, miR 17/21 and miR 92a resulted in a significant increase of non-muscularized arteries and decrease of fully muscularized arteries as compared with Anta-control group. Similar decrease in muscularization of pulmonary arteries was also observed in miR treated groups as provided in representative photomicrographs (**Figure 5B**).

### MCT-induced PH in rats

### Effects of miR 17 antagomir (SEQ ID NO. 10) on hemodynamics and right heart hypertrophy

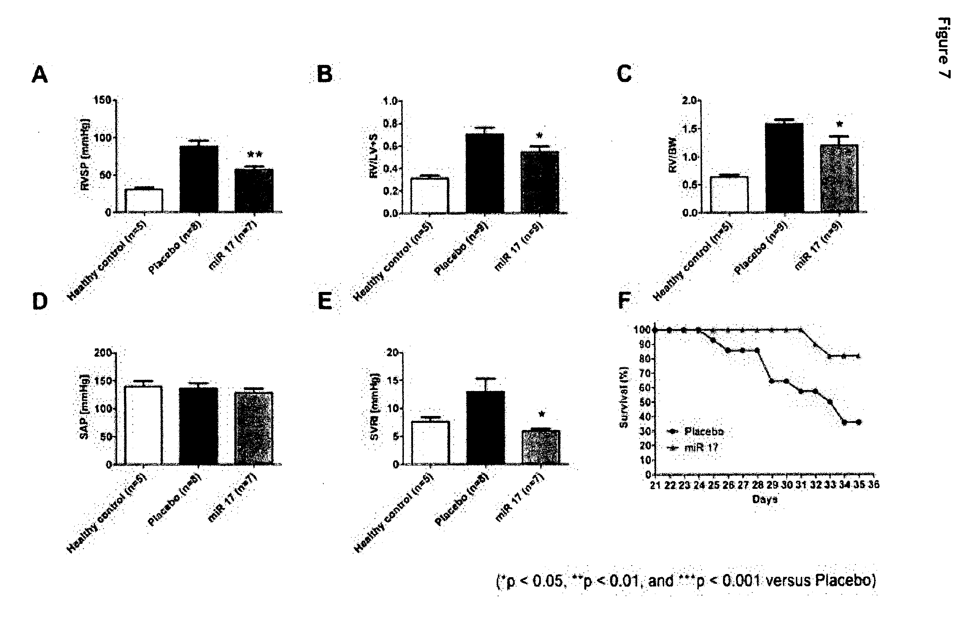

MCT induced a severe pulmonary hypertension in placebo group, as reflected by a significantly increased right ventricular systolic pressure (RVSP) in comparison with healthy control **(****Figure 7A****)**. miR 17 antagomir treatment of rats reduced RVSP, as compared with placebo. The right ventricular hypertrophy was evident from a significantly increased RV/(LV+S)- as well as RVBW- ratio of MCT-injected rats receiving placebo, as compared to the healthy rats. Treatment with miR 17 antagomir significantly reduced the RV/(LV+S)- and RVBW- ratio in comparison with placebo (**Figure 7B****, C**). Mean systemic arterial pressure did not change in any of the treatment groups (**Figure 7D**). However, in the miR 17 antagomir treated rats, systemic vascular resistance index decreased significantly as compared with placebo (**Figure 7E**). Interestingly, miR 17 antagomir treatment improved the survival rate to 80 % as compared to the placebo treated MCT group where survival rate at day 35 was reduced to 30 % (**Figure 7F**).

### Effect of miR 17 antagomir (SEQ ID NO. 10) on acceleration time, cardiac output and right ventricular wall thickness

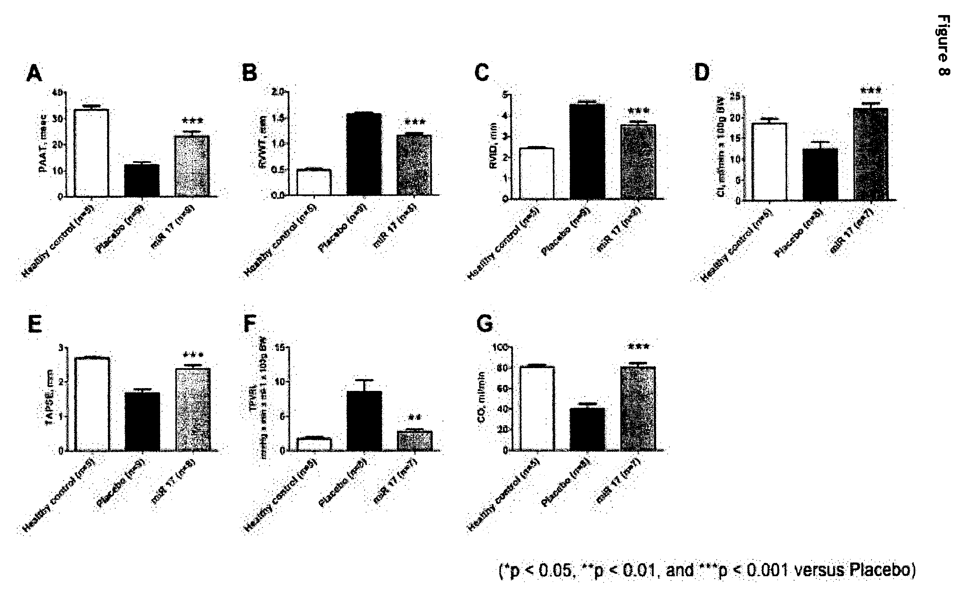

MCT induced a significant decrease of pulmonary artery acceleration time (PAAT) in placebo group, as compared with healthy control. Treatment of the rats with miR 17 antagomir resulted in a noticeably increased PAAT in comparison with placebo group (**Figure 8A**). Right ventricular internal diameter (RVID) and right ventricular wall thickness (RVWT) augmented in rats injected with MCT and chronic treatment with miR 17 antagomir caused the significant decrease of RVID and RVWT, as compared with placebo group (**Figure 8B****, C**). In addition, MCT induced pulmonary hypertension in placebo group as reflected by a decreased cardiac output (CO), tricuspid annular plane systolic excursion (TAPSE) and cardiac index (CI) in comparison with healthy control and chronic treatment with miR 17 antagomir resulted in a significant increase of CO, TAPSE and CI in the treated group, as compared with placebo (**Figure 8D****, E, G**). On the other hand, total pulmonary vascular resistance index (TPVRi) was significantly decreased as compared with placebo (**Figure 8F**).

### Sequences

miRBAse (http://mirbase.org/) ID: hsa-mir-17; miRBAse accession no: MI0000071; SEQ ID NO. 1: 
GUCAGAAUAAUGUCAAAGUGCUUACAGUGCAGGUAGUGAUAUGUGCAUCU
ACUGCAGUGAAGGCACUUGUAGCAUUAUGGUGAC
miRBAse ID: hsa-miR-17; miRBAse accession no: MIMAT0000070; SEQ ID NO. 2: CAAAGUGCUUACAGUGCAGGUAG
miRBAse ID: hsa-mir-21; miRBAse accession no: MI0000077; SEQ ID NO. 3: 
UGUCGGGUAGCUUAUCAGACUGAUGUUGACUGUUGAAUCUCAUGGCAACA
CCAGUCGAUGGGCUGUCUGACA
miRBAse ID: hsa-miR-21; miRBAse accession no: MIMAT0000076; SEQ ID NO. 4: UAGCUUAUCAGACUGAUGUUGA
miRBAse ID: hsa-mir-92a-1; miRBAse accession no: MI0000093; SEQ ID NO. 5: 
CUUUCUACACAGGUUGGAUCGGUUGCAAUGCUGUGUUUCUGUAUGGUAU
UGCACUUGUCCCGGCCUGUUGAGUUUGG
miRBAse ID: hsa-miR-92a; miRBAse accession no: MIMAT0000092; SEQ ID NO. 6: UAUUGCACUUGUCCCGGCCUGU
antisense-17 (RNA), SEQ ID NO. 7:
   CUACCUGCACUGUAAGCACUUUG
antisense-21 (RNA), SEQ ID NO. 8:
   UCAACAUCAGUCUGAUAAGCUA
antisense-92 (RNA), SEQ ID NO. 9:
   CAGGCCGGGACAAGUGCAAUA
Antagomir-17 (mir 17 antagomir) SEQ ID NO. 10:
   CUACCUGCACUGUAAGCACUUUG
      comprising chemical modifications, namely: 3' cholesterol, 2' O-methyl, four consecutive phosphothiolate groups from the 3' side of the molecule, and two consecutive phosphothiolate groups from the 3' side of the molecule (according to Krützfeldt et al., Nature, 2005; 438: 685-689.)
Antagomir-21 (mir 21 antagomir), SEQ ID NO. 11:
   UCAACAUCAGUCUGAUAAGCUA
   comprising chemical modifications, namely: 3' cholesterol, 2' O-methyl, four consecutive phosphothiolate groups from the 3' side of the molecule, and two consecutive phosphothiolate groups from the 3' side of the molecule (according to Krützfeldt et al., Nature, 2005; 438: 685-689.)
Antagomir-92a (mir 92a antagomir), SEQ ID NO. 12:
   CAGGCCGGGACAAGUGCAAUA
   comprising chemical modifications, namely: 3' cholesterol, 2' O-methyl, four consecutive phosphothiolate groups from the 3' side of the molecule, and two consecutive phosphothiolate groups from the 3' side of the molecule (according to Krützfeldt et al., Nature, 2005; 438: 685-689.)

### Figures

**Figure 1** depicts the experimental protocol according to which the results shown in figures 2 and 3 were obtained.
**Figure 2****:** Effects of miR 17, miR 21 miR 17/21 and miR 92a on right ventricular systolic pressure (RVSP), on systemic arterial pressure (SAP), and body weight in chronic hypoxia-induced pulmonary hypertension.
   In mice exposed to chronic hypoxia, miR 17 antagomirs (SEQ ID NOs. 10), miR 21 antagomirs (SEQ ID NOs. 11), miR 17/21 antagomirs (SEQ ID NOs. 10, 11), and miR 92a antagomirs (SEQ ID NO. 12) were administered by tail vein injection from day 14 to 28 of hypoxic exposure followed by hemodynamic measurements. **(A)** Right ventricular systolic pressure (RVSP, in mmHg) is shown (n = 10), **(B)** Systemic arterial pressure (SAP, in mmHg) is shown (n = 10), **(C)** Bodyweight (g) is shown (n = 10). Each bar represents Mean ± SEM. *P<0.05 versus Anta-control; *P<0.001 versus Anta-control.
**Figure 3****:** Effect of miR 17, miR 21 miR 17/21 and miR 92a on right ventricular hypertrophy [RV/(LV+S)] in chronic hypoxia-induced pulmonary hypertension.
   In mice exposed to chronic hypoxia, miR 17 antagomirs (SEQ ID NOs. 10), miR 21 antagomirs (SEQ ID NOs. 11), and miR 92a antagomirs (SEQ ID NO. 12) were administered by tail vein injection from day 14 to 28 of hypoxic exposure followed by hemodynamic right ventricular hypertrophy measurements as described in methods. Right ventricle to left ventricle plus septum weight ratio (RV/LV+S) is shown (n = 10). Each bar represents Mean ± SEM. *P<0.001 versus Anta-control.
**Figure 4****:** Pulmonary artery acceleration time (PA AcT) and cardiac output (CO) measured by high performance echocardiography.
   In mice exposed to chronic hypoxia, miR 17, miR 21 miR 17/21 and miR 92a antagomirs were administered by tail vein injection from day 14 to 28 of hypoxic exposure followed by pulmonary artery acceleration time (PA AcT) **(A)** and cardiac output (CO) **(B)** by high performance ultrasound biomicroscopy. (n = 10). Each bar represents Mean ± SEM. *P<0.05 versus Anta-control; *P<0.001 versus Anta-control.
**Figure 5****:** Effect of miR 17, miR 21 miR 17/21 and miR 92a on the muscularization of small pulmonary arteries of chronic hypoxic mice.
   In mice exposed to chronic hypoxia, miR 17 antagomirs (SEQ ID NOs. 10), miR 21 antagomirs (SEQ ID NOs. 11), miR 17/21 antagomirs (SEQ ID NOs. 10, 11), and miR 92a antagomirs (SEQ ID NO. 12) were administered by tail vein injection from day 14 to 28 of hypoxic exposure. Upon completion of the hemodynamic measurements, the lung tissues were processed for histology. The lung sections were immunostained for von Willebrand factor and a-smooth muscle actin for further morphometric analysis of pulmonary vessels. The proportion of non-, partially or fully muscularized pulmonary arteries (sized 20-70 µm) are given. Results are presented from normoxic as well as chronically hypoxic mice (treated and control groups). Each bar represents Mean ± SEM (n = 10).
**Figure 6** depicts the experimental protocol according to which the results shown in figures 5 and 6 were obtained: Adult male Sprague-Dawley rats (300-350 g BW) were given *s.c.* injection of saline or MCT (60 mg/kg BW). MCT-injected rats were randomized into two groups and were treated with antagomirs of miR 17 and placebo (PBS). All groups of rats treated intravenously (tail vein) day 22 and day 29 with antagomirs of miR 17 and placebo (PBS). Echocardiographic measurement of acceleration time (AcT), cardiac output (CO), heart rate (HR), right ventricular dimensions (RVD) and right ventricular wall thickness (RVWT) was performed at 21st, 28th and 35th day after monocrotaline injection for all experimental groups. At the end of experiments, rats were sacrificed for hemodynamic and right heart hypertrophy (RV/(LV+S) ratio) measurement.
**Figure 7** shows effects of miR 17 antagomir (SEQ ID NO. 10) on hemodynamics: Physiological measurements were taken 35 days after MCT injection. miR 17 antagomir or placebo (PBS) were applied by tail vein injection from day 22 to day 29. (A) Right ventricular systolic pressure (RVSP), in mmHg, (B) measurement of RV hypertrophy [RV/(LV+S)], (C) pulmonary vascular resistance index (PVRI), in mm Hg (D) systemic vascular resistance index (SVRI, in mm Hg (E) cardiac index (CI), in ml/min, (F) systemic arterial pressure (SAP), in mmHg.
**Figure 8** shows miR 17 antagomir effects were measured by high resolution echocardiography: Echocardiography measurements were taken 35 days after MCT injection. miR 17 antagomir or placebo (PBS) were applied by tail vein injection from day 22 to day 29. (A) Right ventricular wall thickness (RVWT, mm), (B) cardiac output (CO, ml/min), (C) total pulmonary vascular resistance index (TPVRi, mmHg x min x ml⁻¹x 100g BW) (D) cardiac index (CI, ml/min), (E) pulmonary artery acceleration time (PAAT, msec) and (F) right ventricular dimensions (RVD, mm).

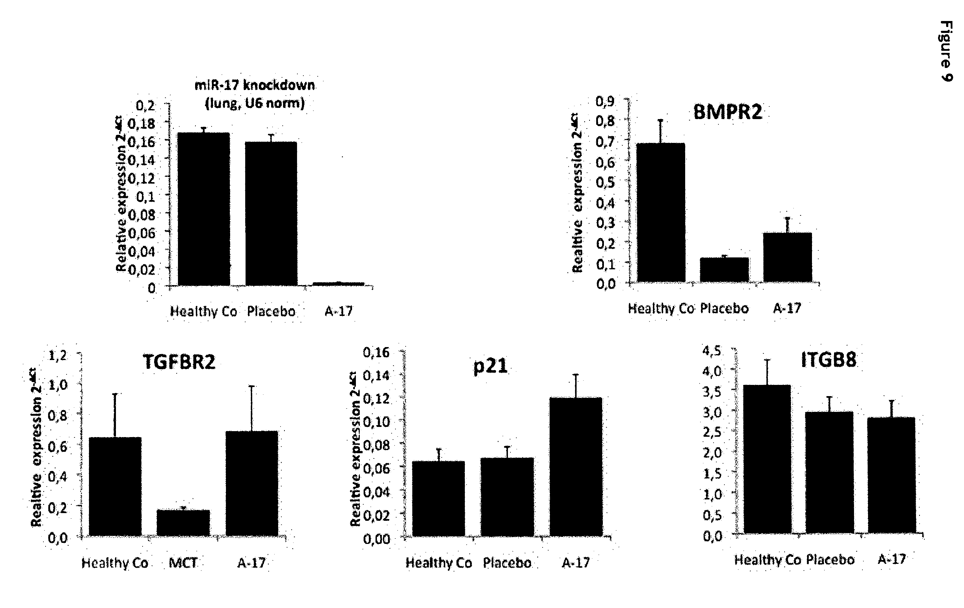

**Figure 9****:** Antagomir-17 (SEQ ID NO. 10) treatment reduces miR 17 (SEQ ID NO. 2) in the lung and affects expression of targets; antogomir-17 affects the cell cycle inhibitor p21 in pulmonary ECs, SMCs, and fibroblasts

BMPR2: bone morphogenetic protein receptor, type II; TGFBR2: transforming growth factor, beta receptor II; CDKN1A: cyclin-dependent kinase inhibitor 1A (p21); ITGB8: integrin B8; EFNB 1: ephrin B1.

## Claims

1. Use of an inhibitor molecule that inhibits the activity of mirR21, miR17, or miR92a in a cell, for treating pulmonary hypertension.

2. A pharmaceutical composition for treating pulmonary hypertension comprising an inhibitor molecule for inhibiting the activity of mirR21, miR17, or miR92a in a cell.

3. A method for treating pulmonary hypertension, comprising introducing an inhibitor molecule for inhibiting the activity of mirR21, miR17, or miR92a into a cell.

4. The use of claim 1, the pharmaceutical composition of claim 2, and the method of claim 3, wherein the inhibitor molecule is a nucleic acid, in particular an oligonucleotide.

5. The use of claim 4, the pharmaceutical composition of claim 4, and the method of claim 4, wherein the nucleic acid molecule is perfectly complementary to mirR21, miR17, or miR92a and comprises a mispairing base at a cleavage site of Ago2 or a base modification for inhibiting cleavage by Ago2.

6. The use of claims 4 or 5, the pharmaceutical composition of claims 4 or 5, and the method of claims 4 or 5, wherein the nucleic acid molecule is selected from the group consisting of antisense DNA-oligonucleotides, RNA-oligonucleotides, antisense DNA-RNA-oligonucleotides antisense 2'-O-methyl oligoribonucleotides, antisense oligonucleotides comprising a phosphorothiaote linkage, antisense oligonucleotides comprising a Locked Nucleic Acid base, morpholino antisense oligonucleotides, and antagomirs.

7. The use of claims 4 to 6, the pharmaceutical composition of claims 4 to 6, and the method of claims 4 to 6, wherein the nucleic acid is complementary to at least part of a nucleotide sequence present in a human microRNA sequence selected from the group consisting of:
miR21 UAGCUUAUCAGACUGAUGUUGA (SEQ ID NO. 4),
miR17 CAAAGUGCUUACAGUGCAGGUAG (SEQ ID NO. 2), and
miR92a UAUUGCACUUGUCCCGGCCUGU (SEQ ID NO 6).

8. The use of claims 4 to 7, the pharmaceutical composition of claims 4 to 7, and the method of claims 4 to 7, wherein the nucleic acid is antisense-17, antisense-21, or antisense-92a with a sequence according to SEQ ID NO. 7, 8, and 9, respectively.

9. The use of claims 4 to 7, the pharmaceutical composition of claims 4 to 7, and the method of claims 4 to 7, wherein the nucleic acid is antagomir-17, antagomir-21 or antagomir-92a with a sequence according to SEQ ID NO. 10, 11, and 12, respectively.

10. The method of claims 3 to 9, wherein the inhibitor molecule is introduced into the cell using
- a physical method, such as microinjection or electroporation;
- a chemical method, such as potassium phosphate transfection; or
- a vector, such as a plasmid or a virus.

11. The use of claims 1 and 4 to 9, the pharmaceutical composition of claims 2 and 4 to 9, and the method of claims 3 to 10, wherein the cell is a mammalian cell, in particular a human cell, in particular a human vascular lung cell.

12. The pharmaceutical composition of claims 2, 4 to 9, and 11, further comprising a pharmaceutically acceptable diluent, carrier or adjuvant.
